# EUROPÄISCHE PATENTANMELDUNG

(11) **EP 2 316 432 A1**
(43) Veröffentlichungstag der Anmeldung: **04.05.2011**
(21) Anmeldenummer: 09013696.1
(22) Anmeldetag: 30.10.2009
(51) Int. Cl.: A61K 9/20, A61K 31/222

(54) **Zusammensetzung enthaltend Fesoterodin und Ballaststoffe**

(71) Anmelder: ratiopharm GmbH, 89079 Ulm (DE)
(72) Erfinder: Stumm, Daniela, 83730 Fischbachbau (DE); Rimkus, Katrin, 80798 München (DE)
(74) Vertreter: Aechter, Bernd

(57) **Zusammenfassung**

Die Erfindung betrifft eine pharmazeutische Zusammensetzung, enthaltend (a) Fesoterodin und/oder Fesoterodin-Metabolite und (b) Ballaststoffe, wobei das Gewichtsverhältnis der Komponenten (a) : (b) im Bereich von 1 : 50 bis 1 : 2 liegt; sowie orale Darreichungsformen, enthaltend die pharmazeutische Zusammensetzung. Ferner betrifft die Erfindung Trockenverfahren zur Herstellung dieser Darreichungsformen.

## Beschreibung

Die Erfindung betrifft eine pharmazeutische Zusammensetzung, enthaltend (a) Fesoterodin und/oder Fesoterodin-Metabolite und (b) Ballaststoffe, sowie orale Darreichungsformen enthaltend die pharmazeutische Zusammensetzung. Ferner betrifft die Erfindung Trockenverfahren zur Herstellung dieser Darreichungsformen.

Fesoterodin ist ein Antimuskarinikum zur Behandlung der überaktiven Blase. Unter Fesoterodin wurden die von den Patienten als sehr belastend empfundenen Symptome der überaktiven Blase deutlich verbessert. In allen klinisch relevanten Endpunkten beider Phase-III-Studien (2, 3) (Dranginkontinenzereignisse/24 h, Miktionshäufigkeit, medianes Miktionsvolumen) ließen sich statistisch signifikante Verbesserungen gegenüber Placebo erzielen. Fesoterodin wird derzeit unter dem Handelsnamen Toviaz^{®} vermarktet.

Der IUPAC-Name von Fesoterodin [INN] ist 2-[(1R)-3-(düsopropylamin)-1-phenylpropyl]-4-(hydroxymethyl)phenyl-isobutyrate. Die chemische Struktur von Fesoterodin wird in nachstehender Formel (1) dargestellt:

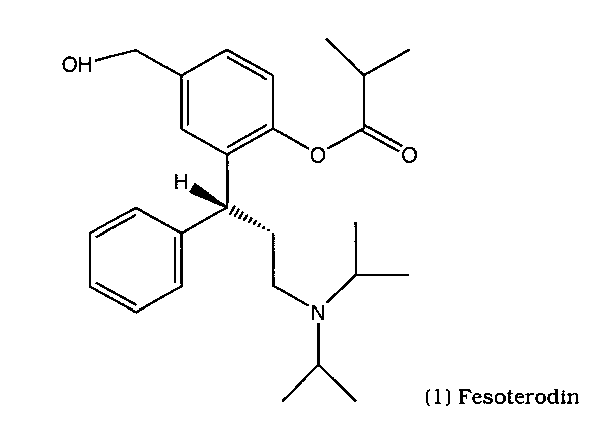

Synthesewege für Fesoterodin sind aus EP 1 077 912 B1 ableitbar. Salze von Fesoterodin sind in EP 1 230 209 B1 beschrieben.

Fesoterodin ist nicht besonders hydrolysestabil. Diesem Umstand Rechnung tragend wurden in WO 2007/141298 Fesoterodin-Tablettenformulierungen vorgeschlagen, die einen Wirkstoff und einen Stabilisator gegen Hydrolyse enthalten, wobei der Stabilisator bevorzugt Xylitol ist. Weiterhin musste der Wirkstoff in eine Matrix aus künstlichem Polymer eingebunden werden, damit eine verlängerte Freisetzung erreicht werden konnte. Es wurde zudem gefunden, dass die Menge an Zersetzungsprodukten nur dann vorteilhaft war, wenn die vorgeschlagenen Formulierungen mittels klassischer Feuchtgranulation hergestellt wurden. Eine Direktverpressung oder auch eine Trockengranulation führte bei identischer Zusammensetzung zu deutlich höheren Mengen an unerwünschten Zersetzungsprodukten (verglichen mit der Feuchtgranulation).

Die im Stand der Technik beschriebenen Herstellverfahren bevorzugen somit ein klassisches Nassgranulierverfahren. Dieses ist wirtschaftlich jedoch aufwendig und sollte vermieden werden. Ferner kommt der Wirkstoff üblicherweise bei der Nassgranulation für längere Zeit in Kontakt mit Lösungsmitteln. Auch dies sollte vermieden werden.

Die im Stand der Technik vorgeschlagenen Formulierungen benötigen des Weiteren unterschiedliche Typen von Hilfsstoffen (einerseits Xylitol, anderseits retardierende Polymere) für Feuchteschutz und Retardierung. Ebenfalls sind mehrere Arbeitsschritte zur Herstellung nötig. Aufgabe der vorliegenden Erfindung war es hingegen, eine Formulierung bereit zu stellen, wobei Hydrolyseschutz und Retardierung mit möglichst einem Hilfsstofftyp und mit möglichst einem Arbeitsschritt erreicht werden können.

Um die gewünschte retardierte Freisetzung zu erreichen, benötigen die im Stand der Technik vorgeschlagenen Formulierungen eine hohe Menge an Polymer. Dadurch wird nur ein relativ niedriger Wirkstoffgehalt (drug load) ermöglicht. So zeigen die in WO 2007/141298 beschriebenen Formulierungen eine Fesoterodingehalt von 5 Gew.-% oder weniger. Eine weitere Aufgabe der Erfindung war es daher, Fesoterodin in einer Form bereit zu stellen, die auch eine Formulierung mit einem hohen Wirkstoffgehalt, bevorzugt mit einem Wirkstoffgehalt von mehr als 5 %, ermöglicht.

Problematisch bei den im Stand der Technik beschriebenen Tabletten war ferner, dass ein erheblicher Teil des Wirkstoffes (ca. 20 %) in der Regel nicht freigesetzt wird. Eine Aufgabe der vorliegenden Erfindung war es daher, eine Darreichungsform mit modifizierter Freisetzung bereit zu stellen, wobei der Wirkstoff möglichst vollständig freigesetzt werden sollte.

Fesoterodin wird zur Behandlung der überaktiven Blase verwendet. Diese Indikation erfordert es, dass Patienten die Darreichungsformen stets mit sich führen. Die derzeit vermarkteten Toviaz^{®} Tabletten weisen jedoch nur eine Lagerstabilität bis 25 °C auf. Dies ist insbesondere in den Sommermonaten unbefriedigend. Eine weitere Aufgabe der Erfindung war es daher, Fesoterodin in einer Form bereit zu stellen, die für eine Formulierung mit einer Lagerstabilität im praktischen Gebrauch von bis zu 30 °C geeignet ist.

Zudem war es Aufgabe der Erfindung, eine pharmazeutische Darreichungsform zur Behandlung der überaktiven Blase bereit zu stellen, die im Wesentlichen eine gleiche Löslichkeit wie die in WO 2007/141298 dargestellten Formulierungen, insbesondere die in Tabelle 1 dargestellten Beispielsformulierungen, aufweist und in Folge bei einer oralen Verabreichung im Wesentlichen bioäquivalent dazu ist.

Schließlich ist aus toxikologischer Sicht festzustellen, dass Fesoterodin ein sehr aktiver Wirkstoff ist, da er im Körper durch unspezifische Esterasen schnell und weitgehend vollständig aktiviert wird. Somit war es Aufgabe der Erfindung, eine "sichere" Fesoterodin-Formulierung bereit zu stellen, wobei ein zu schnelles Anfluten verhindert wird.

Die vorstehend genannten Aufgaben konnten unerwartet durch Kombination von Fesoterodin mit Ballaststoffen gelöst werden.

Gegenstand der Erfindung ist daher eine pharmazeutische Zusammensetzung, enthaltend
(a) Fesoterodin und/oder Fesoterodin-Metabolite, und
(b) Ballaststoffe,
wobei das Gewichtsverhältnis der Komponenten (a) : (b) im Bereich von 1 : 50 bis 1 : 2 liegt.

Gegenstand der Erfindung ist ferner ein Verfahren zur Herstellung von oralen Darreichungsformen, insbesondere von Tabletten, umfassend die Schritte:
(i) Vermischen von
   a) Fesoterodin und/oder Fesoterodin-Metabolite,
   b) Ballaststoffen mit pharmazeutischen Hilfsstoffen,
   und gegebenenfalls weiteren pharmazeutischen Hilfsstoffen;
(ii) Kompression zu Tabletten, gegebenenfalls unter Zusatz weiterer pharmazeutischer Hilfsstoffe; und
(iii) gegebenenfalls Befilmung der Tabletten.

Ebenfalls sind Tabletten, erhältlich durch das erfindungsgemäße Verfahren, Gegenstand dieser Erfindung.

Schließlich ist Gegenstand der Erfindung die Verwendung von Ballaststoffen zur Herstellung einer pharmazeutischen Formulierung mit modifizierter Freisetzung zur Behandlung der überaktiven Blase.

Fesoterodin ist ein Prodrug. Nach oraler Einnahme erfolgt durch Esterasen im menschlichen Körper eine Aktivierung des Prodrugs zum aktiven Metaboliten. Die vorliegende Erfindung betrifft im Allgemeinen Fesoterodin und dessen Metabolite. Der Ausdruck "Fesoterodin" bezieht sich daher im Rahmen der vorliegenden Anmeldung grundsätzlich auf Fesoterodin und/oder dessen Metabolite. Unter Metabolite werden in diesem Zusammenhang alle Substanzen verstanden, die bei der Verstoffwechselung von Fesoterodin entstehen, insbesondere bei der Verstoffwechselung im menschlichen Körper entstehen.

Bevorzugt handelt es sich bei den Metaboliten um Fesoterodin-5-HM gemäß der nachfolgenden Struktur (2):

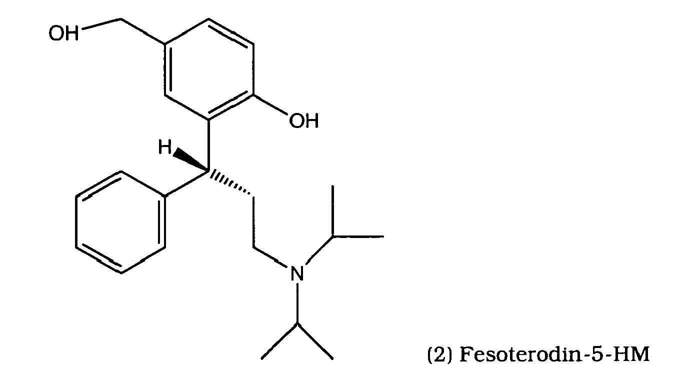

Da sich im Rahmen dieser Anmeldung die Erläuterungen zum Wirkstoff üblicherweise sowohl auf Fesoterodin als auch auf Fesoterodin-Metabolite beziehen, wird häufig auch der Ausdruck "Fesoterodin(metabolit)" verwendet. Grundsätzlich umfasst im Rahmen dieser Anmeldung der Begriff "Fesoterodin" oder "Fesoterodin-Metabolit" sowohl die vorstehend in den Strukturen (1) und (2) dargestellte "freie Base" als auch pharmazeutisch verträgliche Salze davon. Hierbei kann es sich um ein oder mehrere Salze handeln, die auch im Gemisch vorliegen können. Unter "Salz" wird hierbei verstanden, dass die Amingruppe des Fesoterodins oder des Fesoterodin-Metabolits protoniert wurde, wobei sich ein positiv geladenes Stickstoffatom bildet, das mit einem entsprechenden Gegenanion assoziiert ist. Die entsprechenden Salze werden im Rahmen dieser Anmeldung auch als "Fesoterodin(metabolit)salze" bezeichnet.

Bevorzugt werden als Salze Säureadditionssalze verwendet. Beispiele für geeignete Salze sind Hydrochloride, Carbonate, Hydrogencarbonate, Acetate, Lactate, Butyrate, Propionate, Sulfate, Methansulfonate, Citrate, Fumarat, Hydrogenfumarat, Tartrate, Maleinat, Nitrate, Sulfonate, Oxalate und/oder Succinate.

Besonders bevorzugt handelt es sich im Fall von Fesoterodin oder Fesoterodin-Metabolit bei dem pharmazeutisch verträglichen Salz um Hydrogenfumarat. Hydrogenfumarat ist eine Verbindung gemäß der Formel HOOC-CH=CH-COO wobei die Doppelbindung eine E-Konfiguration aufweist. Weiterhin besonders bevorzugt handelt es sich im Falle von Fesoterodin oder Fesoterodin-Metabolit bei dem pharmazeutisch verträglichen Salz um Fumarat. Fumarat ist eine Verbindung gemäß der Formel OOC-CH=CH-COO. wobei die Doppelbindung eine E-Konfiguration aufweist.

Ebenfalls besonders bevorzugt handelt es sich bei dem pharmazeutisch verträglichen Salz um Tartrat, d.h. um ein Weinsäuresalz. Weinsäure ist im Fachgebiet auch als 2,3-Dihydroxybernsteinsäure bekannt. Im Rahmen dieser Erfindung kann Weinsäure als D-(-)-Weinsäure, L-(+)-Weinsäure, meso-Weinsäure oder ein beliebiges Gemisch davon, z.B. als DL-Racemat, eingesetzt werden. 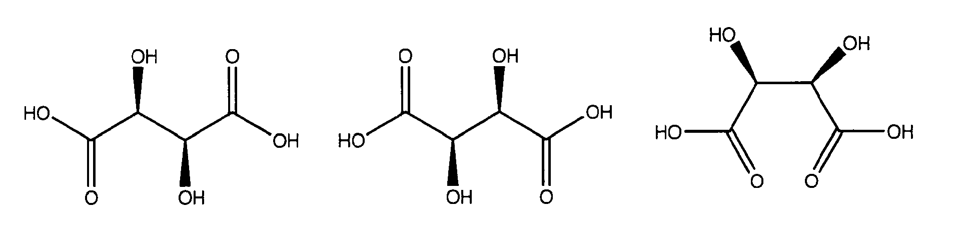 D-(-)-Weinsäure L-(+)-Weinsäure meso-Weinsäure In einer bevorzugten Ausführungsform wird L-(+)-Weinsäure verwendet.

Im erfindungsgemäßen Fesoterodin(metabolit)salz kann Weinsäure als zweifach (Tartrat) oder einfach (Hydrogentartrat) negativ geladenes Anion vorliegen. Bevorzugt liegt die Weinsäure als Tartrat vor. Entsprechend ist es möglich, dass das molare Verhältnis von Fesoterodin(metabolit) zu Weinsäure 1 : 1 bis 2 : 1 beträgt. Bevorzugt beträgt im erfindungsgemäßen Fesoterodin(metabolit)salz das molare Verhältnis von Fesoterodin(metabolit) zu Weinsäure etwa 2:1.

Grundsätzlich kann das erfindungsgemäße Fesoterodin(metabolit)salz beispielsweise in amorpher Form, kristalliner Form oder in Form einer festen Lösung vorliegen. Bevorzugt liegt das erfindungsgemäße Fesoterodin(metabolit)salz in kristalliner Form vor.

Somit wird im Rahmen dieser Erfindung bevorzugt Fesoterodin-Hydrogenfumarat, Fesoterodin-Fumarat, Fesoterodin-Tartrat, Fesoterodin-5-HM-Hydrogenfumarat, Fesoterodin-5-HM-Fumarat, Fesoterodin-5-HM-Tartrat, Fesoterodin-5HM-Hydrogentartrat oder Gemische davon als Wirkstoff verwendet. Insbesondere wird Fesoterodin-Fumarat verwendet. Insbesondere wird Fesoterodin-5-HM-Tartrat verwendet.

Bevorzugt wird für den Kern Fesoterodin und/oder Fesoterodin-Metabolit mit einem Wassergehalt von 0,1 bis 5 Gew.-%, mehr bevorzugt von 0,3 bis 3 Gew.-%, verwendet. Der Wassergehalt wird gemäß coulometrischer Karl-Fischer Titration bestimmt und bevorzugt mittels "Oven Sample Processor 774" wie in Metrohm, Application Bulletin 280/1d, beschrieben.

Unter Ballaststoffe (b) werden im Allgemeinen Stoffe verstanden, die üblicherweise in Lebensmitteln enthalten sein können, aber im Gastrointestinaltrakt nicht verdaulich sind. Es kann sich um natürliche oder synthetische Ballaststoffe handeln. Natürliche Ballaststoffe sind bevorzugt. Unter "natürlich" werden hierbei Ballaststoffe auf Basis von natürlich vorkommenden Bausteinen verstanden, wobei die Bausteine chemisch modifiziert sein können.

Ein Beispiel für geeignete synthetische Ballaststoffe sind Ionenaustauscherharze. Ein Ionenaustauscherharz ist ein Polymer, mit dem gelöste Ionen gegen andere Ionen gleicher Ladungsart ersetzt werden können. Mehr bevorzugt wird ein Kationenaustauscherharz verwendet. Unter Kationenaustauscherharz ist ein Polymer zu verstehen, das funktionelle Gruppen mit einem abdissoziierbaren Kation enthält. Beispiele für diese funktionellen Gruppen sind Sulfonsäuregruppen/Sulfonatgruppen oder Carboxylgruppen/Carboxylatgruppen. Somit wird als synthetischer Ballaststoff (b) bevorzugt ein Polymer verwendet, das Carboxylgruppen/Carboxylatgruppen und/oder Sulfonylgruppen/Sulfonatgruppen enthält. Sofern Carboxylat- oder Sulfonatgruppen vorliegen, können z.B. Ammonium-, Alkali- und Erdalkaliionen als Gegenionen dienen, bevorzugt sind Natrium und Kalium, insbesondere Kalium.

In einer bevorzugten Ausführungsform handelt es sich bei dem synthetischen Ballaststoff (b) um ein Copolymer, erhältlich durch Copolymerisation von Methacrylsäure und Divinylbenzol. Ein derartiges Copolymer ist unter der Bezeichnung Polacrilin bekannt. Insbesondere wird im Rahmen dieser Erfindung Polacrilin in Form des Kaliumsalzes (Polacrilin-Kalium, insbesondere gemäß US Pharmacopoeia monographiert) verwendet.

Polacrilin-Kalium kann durch folgende Strukturformel veranschaulicht werden. 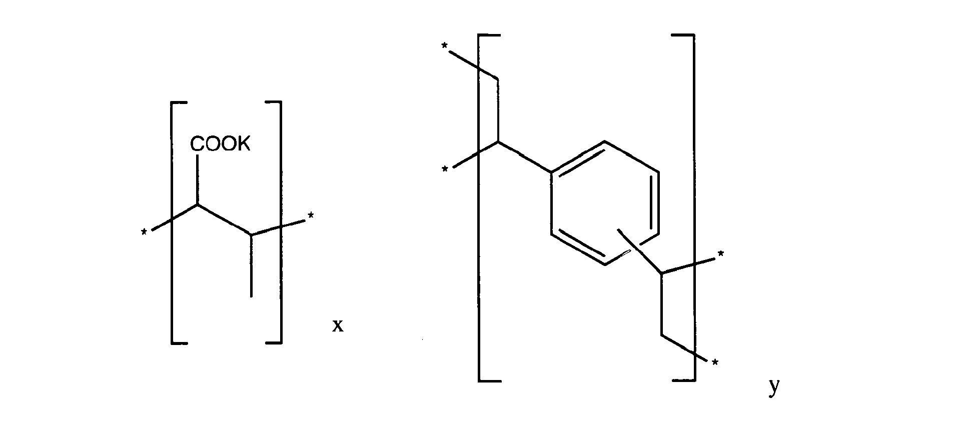 wobei x und y natürliche Zahlen sind, beispielsweise von 10¹ bis 10²⁰, bevorzugt von 10⁶ bis 10¹⁸. Das Verhältnis von x zu y beträgt üblicherweise 50 : 1 bis 1 : 1, bevorzugt 20 : 1 bis 2 : 1, besonders bevorzugt 10 : 1 bis 3 : 1.

In einer bevorzugten Ausführungsform bestehen die Ballaststoffe (b) aus natürlichen Ballaststoffen. Bevorzugt handelt es sich hierbei um pflanzliche Ballaststoffe, d.h. um Stoffe, die aus Pflanzen gewonnen werden können. Mehr bevorzugt handelt es sich um pflanzliche Ballaststoffe, die ein Geliervermögen zeigen (d.h. durch Zugabe dieser Ballaststoffe zu Wasser erhöht sich die Viskosität, bevorzugt bildet sich ein Gel.)

In einer bevorzugten Ausführungsform weisen die Ballaststoffe (b) eine Gel-Stärke (im Englischen auch als *"Bloom-Strength"* oder *"Gel-Strength"* bekannt) von 5 bis 500 g, mehr bevorzugt von 30 bis 300 g, noch mehr bevorzugt von 50 bis 250 g, besonders bevorzugt von 60 bis 200 g, insbesondere 80 bis 150 g, auf.

Die "Gel-Stärke" ist ein Maß für die Festigkeit eines Gels, das aus einer 6,67 Gew.-%igen Lösung (bestehend aus Ballaststoffen und Wasser) hergestellt wurde. Die vorstehend angegebenen Werte geben die Masse an, die notwendig ist, um eine definierte Oberfläche eines Gels um 4 mm einzudrücken. Die Gel-Stärke wird im Rahmen dieser Anmeldung gemäß den offiziellen Verfahren des "Gelatin Manufacturers Institute of America" (kurz "GMIA") bestimmt. Hierzu wird auf die "GMIA Standard Methods for The Testing Of Edible Gelatine", September 2006, verwiesen. Hierbei wird ein "LFRA Texture Analyzer" von Brookfield Engineering verwendet, der einen Stempel mit 0.5" (0,5 Zoll) Durchmesser und nicht abgeschrägten Ecken aufweist.

In einer bevorzugten Ausführungsform enthalten die Ballaststoffe (b) keine Cellulose oder Cellulosederivate wie z.B. Celluloseester oder Celluloseether. Insbesondere sind die Ballaststoffe (b) frei von Methylcellulose, Methylethylcellulose, Hydroxyethylcellulose, Hydroxypropylmethylcellulose, Hydroxypropylcellulose oder Carboxymethylcellulose oder Mischungen daraus. Ebenfalls enthält auch die erfindungsgemäße Zusammensetzung bevorzugt nicht vorstehend genannte Cellulosederivate oder Cellulose.

Des Weiteren enthält in einer bevorzugten Ausführungsform die Komponente (b), oder mehr bevorzugt die pharmazeutische Zusammensetzung, kein Polyvinylpyrrolidon, pregelatinierte Stärke, Polymethacrylat, Polyvinylacetat, Dextran, Stärke oder Gemische daraus.

In einer besonders bevorzugen Ausführungsform sind die Ballaststoffe (b) ausgewählt aus Alginat, Gelatine, Agar, Gummi Arabicum, Traganth, Xanthan und Carrageenan. Insbesondere wird als Ballaststoff (b) Kappa-Carrageenan verwendet.

Die einzelnen Ballaststoffe (b) werden nachfolgend näher erläutert.

### Agar:

Agar (E-406) findet sich üblicherweise in der Zellwand von Rotalgen (Rhodophyceae), meist in Form von Calcium- und Magnesiumsalzen. Die Herstellung erfolgt üblicherweise durch Heisswasserextraktion, Reinigung und anschließende Trocknung.

Agar enthält bevorzugt zwei Fraktionen: Agarose und Agaropektin. Der Agaroseanteil beträgt üblicherweise 40 bis 75 Gew.-%, bevorzugt 55 bis 66 Gew.-% des Gesamtgewichts. Der Agaroseanteil ist im Wesentlichen für das Geliervermögen verantwortlich. Es handelt sich im Allgemeinen um ein neutrales kettenförmiges Polysaccharid, in dem D-Galactose und 3,6-Anhydro-L-Galactose abwechselnd in β-1,4- und α-1,3-glycosidischer Bindung miteinander verknüpft sind. Agaropektin hat üblicherweise die gleiche Basisstruktur wie Agarose, enthält aber bis zu 10 % Sulfatgruppen, D-Glucuronsäure und gegebenenfalls Brenztraubensäure.

Bevorzugt wird Agar mit einem gewichtsmittleren Molekulargewicht von 5000 bis 160.000 g/mol, mehr bevorzugt von 10.000 bis 130.000 g/mol verwendet. Im Rahmen dieser Erfindung wird das gewichtsmittlere Molekulargewicht mittels GelPermeationschromatographie bestimmt.

Agar ist ein besonders bevorzugter Ballaststoff (b).

### Alginate:

Alginate (E-401) sind Salze der Alginsäure (E-400). Alginsäure ist bevorzugt ein lineares Polysaccharid, aufgebaut aus D-Mannuronsäure und L-Guluronsäure, die β-1,4-glycosidisch miteinander verknüpft sind. Bevorzugt werden Ammoniumalginat (E-403), Calciumalginat (E-404), Kaliumalginat (E-402) und/oder Natriumalginat (E-401) verwendet. Die Gewinnung erfolgt üblicherweise aus Tange (Kelp) - hauptsächlich werden Macrocystis pyrifera und Laminaria-Arten - verwendet. Die Alginsäure wird üblicherweise mit Alkali extrahiert und anschließend werden im Sauren entsprechende Salze ausgefällt.

Bevorzugt werden Alginate mit einem gewichtsmittleren Molekulargewicht von 20.000 bis 240.000 g/mol, mehr bevorzugt von 30.000 bis 180.000 g/mol, verwendet.

### Carrageenan:

Als Carrageenan (E-407) werden üblicherweise die - bevorzugt gereinigten und getrockneten - Extrakte aus rotem Seetang (Rhodophyceae) bezeichnet. Die zur Gewinnung von Carrageenan verwendeten Gattungen sind bevorzugt Chondrus *crispus, Gigartina stellata* und in zunehmendem Masse *Eucheuma cottonii* sowie *Eucheuma spinosa.* In getrockneter Form werden diese Rohstoffe auch Carrageen (Irländisches Moos) genannt.

Zur Herstellung werden bevorzugt die gereinigten Rotalgen mit heißem Wasser oder alkalisch extrahiert. Der Extrakt wird entweder direkt getrocknet oder zur Ausfällung des Carrageenans mit Alkohol versetzt.

Bevorzugte Ausführungsformen von Carrageenan sind Lambda(λ)-Carrageenan, Kappa(κ)-Carrageenan und Iota(t)-Carrageenan.

Lambda-Carrageenan ist ein Kettenmolekül, das aus dimeren Bausteinen, β-D-Galactosido(1,4)-α-D-Galactose, aufgebaut ist. Diese Dimere sind dabei 1,3-glycosidisch miteinander verknüpft. Die primäre Alkoholgruppe der α-D-Galactose ist bevorzugt mit Schwefelsäure verestert. Die Hydroxygruppen am C-2 beider Galactosen sind, bevorzugt bis zu etwa 70 %, mit Schwefelsäure verestert. Lambda-Carrageenan weist bevorzugt einen Sulfatgehalt zwischen 25 und 45 %, mehr bevorzugt zwischen 32 und 39 %, auf.

Lambda-Carrageenan weist bevorzugt folgende Struktureinheit auf:

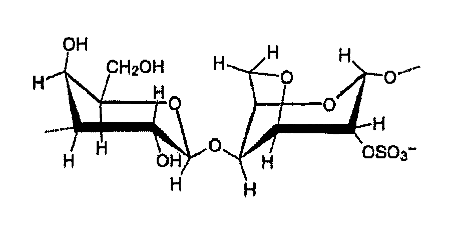

Kappa-Carrageenan ist üblicherweise aus dem Dimer Carrabiose aufgebaut, in welchem β-D-Galactose 1,4-glycosidisch an α-D-3,6-Anhydrogalactose gebunden ist. Diese Dimere sind durch 1,3-glycosidische Bindungen zu einem Kettenmolekül verknüpft. Üblicherweise ist K-Carrageenan teilweise sulfatiert, es befindet sich bevorzugt eine Sulfatester-Gruppe am C-4 der Galactose; Kappa-Carrageenan weist bevorzugt einen Sulfatgehalt zwischen 20 und 35 %, mehr bevorzugt zwischen 25 und 30 %, auf.

Kappa-Carrageenan weist bevorzugt folgende Struktureinheit auf:

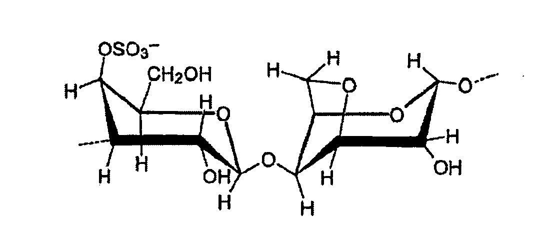

Kappa-Carrageenan ist im Rahmen dieser Erfindung als Ballaststoff (b) bevorzugt.

Die Struktur von Iota-Carrageenan entspricht im Wesentlichen der des Kappa-Carrageenans, wobei zusätzlich die Hydroxygruppe am C-2 der Anhydrogalactose mit Schwefelsäure verestert sein kann. Der Sulfatgehalt liegt üblicherweise zwischen 28 und 35 %.

Iota-Carrageenan weist bevorzugt folgende Struktureinheit auf:

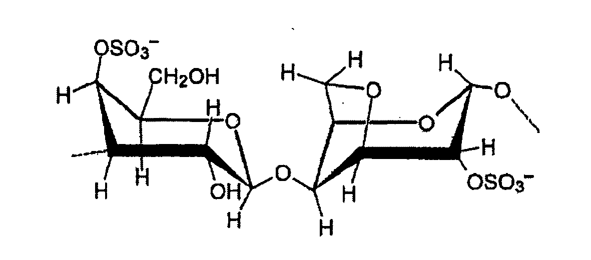

Bevorzugt werden Carrageenane mit einem gewichtsmittleren Molekulargewicht von 80.000 bis 850.000 g/mol, mehr bevorzugt von 120.000 bis 750.000 g/mol, verwendet.

Carrageenane können in Form von Salzen, z.B. in Form von Kalium-, Natrium- oder Calciumsalzen vorliegen.

### Gelatine:

Gelatine wird üblicherweise durch selektive Hydrolyse des Collagens, (eines Bestandteils des Bindegewebes von tierischen Häuten und Knochen) erhalten. Als Ausgangsmaterial werden beispielsweise Knochen, Hautstücke und Schweineschwarten verwendet. Die Rohmaterialien werden üblicherweise vorgereinigt und gegebenenfalls entfettet. Knochen werden üblicherweise zusätzlich zu sogenanntem Ossein decalciniert. Anschließend wird üblicherweise das Collagen durch Säure- oder Alkalibehandlung gequollen und die Gelatine in saurem Milieu in der Hitze extrahiert.

Gelatine ist üblicherweise ein lineares Protein, bevorzugt mit amphoterem Charakter. Das gewichtsmittlere Molekulargewicht liegt üblicherweise bei 10.000 bis 100.000, bevorzugt bei 15.000 bis 90.000 g/mol. Gelatine enthält bevorzugt die Aminosäuren Glycin (20 bis 30 %), Prolin (14 bis 24 %), Hydroxyprolin (10 bis 18 %), Alanin (8 bis 16 %), Asparaginsäure (7 bis 14 %), Arginin (6 bis 11 %), Glutaminsäure (4 bis 8 %), Lysin (3 bis 7 %), Leucin (3 bis 7 %) und Serin (2 bis 5 %).

### Gummi Arabicum:

Gummi arabicum (E-414) kann aus Exsudat-Gummi, d.h. aus getrocknetem Pflanzensaft, gewonnen werden. Gummi arabicum ist ein saures, verzweigtes Polysaccharid, das beispielsweise in Form gemischter Kalium-, Magnesium- und Calciumsalze vorliegen kann. Die freie Säure (Arabinsäure) enthält üblicherweise als monomere Bausteine D-Galactose, L-Arabinose, L-Rhamnose, D-Glucuronsäure.

Bevorzugt wird Gummi arabicum mit einem gewichtsmittleren Molekulargewicht von 100.000 bis 400.000 g/mol, mehr bevorzugt von 200.000 bis 300.000 g/mol verwendet.

### Traganth:

Traganth (E-413), kann aus dem Saft von *Astragalus*-Sträuchern gewonnen werden. Traganth ist ein verzweigtes Polysaccharid, enthaltend D-Galacturonsäure, L-Arabinose, D-Galactose, L-Fucose und D-Xylose. Bevorzugt wird Traganth mit einem gewichtsmittleren Molekulargewicht von 500.000 bis 1.00.000 g/mol, mehr bevorzugt von 700.000 bis 900.000 g/mol, verwendet.

### Xanthan:

Xanthan (E-415) ist ein extrazelluläres Polysaccharid mikrobiellen Ursprungs. Seine Gewinnung kann durch Fermentation mittels Xanthomonas *campestris* und anschließender Alkoholfällung des Kulturfiltrates erfolgen. Xanthan enthält D-Glucose, D-Mannose und D-Glucuronsäure, bevorzugt etwa im Verhältnis 2:2:1. Bevorzugt wird Xanthan mit einem gewichtsmittleren Molekulargewicht von 500.000 bis 3.000.000 g/mol, mehr bevorzugt von 800.000 bis 2.000.000 g/mol, verwendet.

Xanthan kann beispielsweise als Natrium-, Calcium- und/oder Kaliumsalz verwendet werden.

Neben den vorstehend genannten Ballaststoffen (b) können ferner Galactomannane verwendet werden. Galactomannane stellen das Endosperm von Samen verschiedener Leguminosenarten dar. Endosperme werden hierbei üblicherweise zu Mehlen vermahlen.

Die bevorzugten Galactomannane (welche sich insbesondere im Verhältnis Mannose/Galactose unterscheiden), sind Johannisbrotkernmehl (Carubin, Locust Bean Gum E-410), bevorzugt Mannose/Galactose ca. 4:1, das bevorzugt aus den Samen der *Ceratonia siliqua* erhältlich ist;
Guarkernmehl (Guaran, GuarGum, E-412), bevorzugt Mannose/Galactose ca. 2:1, das bevorzugt aus den Samen der *Cyamopsis tetragonolobus* und C. *psoralioides* erhältlich ist;
und das Tarakernmehl (Tara, E-417), bevorzugt Mannose/Galactose ca. 3:1, das bevorzugt aus den Samen von *Caesalpinia spinosa* erhältlich ist.

Ferner kann Tamarind als Komponente (b) verwendet werden. Tamarind ist üblicherweise ein aus den Samen der Tamarinde *(Tamarindus indica)* erhältliches Hydrokolloid, enthaltend 1,4-verknüpfte D-Glucose Einheiten in der Hauptkette und D-Xylose, D-Galactose und L-Arabinose in den Verzweigungen. Das gewichtsmittlere Molekulargewicht beträgt bevorzugt 20.000 bis 80.000, mehr bevorzugt 30.000 bis 70.000 g/mol.

Ferner kann Karaya als Komponente (b) verwendet werden. Karaya (E-416) ist ein aus getrockneten Pflanzensäften erhältliches Exsudat-Gummi. Es wird bevorzugt aus *Sterculia* Arten, speziell Sterculia urens oder aus *Cochlospermum,* gewonnen. Karaya enthält acetyliertes Polysaccharid, das insbesondere D-Galactose, L-Rhamnose, D-Galacturonsäure und D-Glucuronsäure umfasst.

Fesoterodin(metabolit) (a) und Ballaststoff (b) werden üblicherweise als partikuläre Feststoffe eingesetzt. Hierbei beträgt der mittlere Teilchendurchmesser (D50) üblicherweise 1 bis 500 *µ*m, bevorzugt 10 bis 250 *µ*m, mehr bevorzugt 15 bis 150 *µ*m, besonders bevorzugt 20 bis 120 *µ*m, insbesondere 25 bis 90 *µ*m. Es ist bevorzugt, dass Fesoterodin (a) und Ballaststoffe (b) eine monomodale Teilchengrößenverteilung bilden, insbesondere im Hinblick auf eine vorteilhafte Gleichförmigkeit des Gehalts.

Der Ausdruck "mittlerer Teilchendurchmesser" bezieht sich im Rahmen dieser Erfindung - sofern nichts anderes angegeben - auf den D50-Wert des volumenmittleren Teilchendurchmessers, der mittels Laserdiffraktometrie bestimmt wurde. Insbesondere wurde zur Bestimmung ein Mastersizer 2000 von Malvern Instruments verwendet (Nassmessung, 2000 rpm, flüssiges Paraffin als Dispergiermittel, Ultraschall 60 sek., Auswertung gemäß der Fraunhofer Methode). Der mittlere Teilchendurchmesser, der auch als D50-Wert der integralen Volumenverteilung bezeichnet wird, wird im Rahmen dieser Erfindung als der Teilchendurchmesser definiert, bei dem 50 Gew.-% der Teilchen einen kleineren Durchmesser haben als der Durchmesser, der dem D50-Wert entspricht. Ebenso haben dann 50 Gew.-% der Teilchen einen größeren Durchmesser als der D50-Wert.

In einer bevorzugten Ausführungsform der Erfindung liegt ein Gewichtsverhältnis der Komponenten (a) : (b) im Bereich von 1 : 50 bis 1 : 2, mehr bevorzugt von 1 : 30 bis 1 : 3, noch mehr bevorzugt von 1 : 20 bis 1 : 4, insbesondere 1 : 15 bis 1 : 5 vor.

Neben Ballaststoffen (b) kann die erfindungsgemäße pharmazeutische Formulierung noch weitere pharmazeutische Hilfsstoffe umfassen. Hierbei handelt es sich um die dem Fachmann bekannten Hilfsstoffe, beispielsweise solche, die im Europäischen Arzneibuch beschrieben sind. Beispiele für verwendete Hilfsstoffe sind Sprengmittel, Tablettiermittel, Trennmittel, Zusätze zur Verbesserung der Pulverfließfähigkeit, Gleitmittel, Netzmittel, und/oder Schmiermittel.

In einer bevorzugten Ausführungsform enthält die erfindungsgemäße pharmazeutische Zusammensetzung
a) 0,1 bis 20 Gew.-%, mehr bevorzugt 0,5 bis 10 Gew.-% Fesoterodin und/oder Fesoterodin-Metabolite;
b) 0,5 bis 80 Gew.-%, mehr bevorzugt 15 bis 60 Gew.-% Ballaststoffe;
c) 0 bis 15 Gew.-%, mehr bevorzugt 0,2 bis 5 Gew.-% Sprengmittel; und
d) 0 bis 80 Gew.-%, mehr bevorzugt 25 bis 75 Gew.-% Tablettiermittel, bezogen auf das Gesamtgewicht der Formulierung.

Die erfindungsgemäße Formulierung kann Sprengmittel enthalten (c). Als Sprengmittel werden im Allgemeinen Stoffe bezeichnet, die den Zerfall einer Darreichungsform, insbesondere einer Tablette, nach Einbringen in Wasser beschleunigen. Geeignete Sprengmittel sind z.B. organische Sprengmittel wie Natriumcarboacymethylstärke, Croscarmellose und Crospovidon. Alternativ verwendet werden alkalische Sprengmittel. Unter alkalischen Sprengmitteln sind Sprengmittel zu verstehen, die beim Lösen in Wasser einen pH-Wert von mehr als 7,0 erzeugen, beispielsweise NaHCO₃ oder Na₂C0₃.

Die erfindungsgemäße Formulierung kann Tablettiermittel (d) enthalten. Unter Tablettiermittel werden Stoffe verstanden, die eine Füllstoffwirkung und/oder ein Bindemittelwirkung aufweisen. Unter Füllstoffe sind im Allgemeinen Stoffe zu verstehen, die zur Bildung des Tablettenkörpers bei Tabletten mit geringen Wirkstoffmengen dienen. Das heißt, Füllstoffe erzeugen durch "Strecken" der Wirkstoffe eine ausreichende Tablettiermasse. Füllstoffe dienen üblicherweise also dazu, eine geeignete Tablettengröße zu erhalten.

Beispiele für bevorzugte Tablettiermittel sind Lactose, Saccharose, mikrokristalline Cellulose (z.B. Avicel^{®}), Stärke, Calciumphosphat, Calciumcarbonat, Magnesiumcarbonat, Magnesiumoxid, Calciumsulfat, hydrogeniertes Pflanzenöl, Dextrin. Cyclodextrin und Kaolin. Ebenfalls kann silifizierte mikrokristalline Cellulose verwendet werden. Die bevorzugt verwendete silifizierte mikrokristalline Cellulose ist kommerziell erhältlich unter dem Handelsnamen Prosolv^{®} und weist einen Siliciumdioxidgehalt von 1 bis 3 Gew.-%, bevorzugt von 2 Gew.-% auf.

Ein Beispiel für einen Zusatz zur Verbesserung der Pulverfließfähigkeit ist disperses Siliciumdioxid, z.B. bekannt unter dem Handelsnamen Aerosff^{®}. Zusätze zur Verbesserung der Pulverfließfähigkeit werden üblicherweise in einer Menge von 0,1 bis 3 Gew.-%, bezogen auf das Gesamtgewicht der Formulierung, verwendet.

Ferner können Schmiermittel verwendet werden. Schmiermittel dienen im Allgemeinen der Verringerung der Gleitreibung. Insbesondere soll die Gleitreibung vermindert werden, die beim Tablettieren einerseits zwischen den sich in der Matrizenbohrung auf und ab bewegenden Stempeln und der Matrizenwand sowie andererseits zwischen Tablettensteg und Matrizenwand besteht. Geeignete Schmiermittel stellen z.B. Stearinsäure, Adipinsäure, Natriumstearylfumarat (bekannt unter dem Handelsnamen Pruv^{®}) und/oder Magnesiumstearat dar.

Schmiermittel werden üblicherweise in einer Menge von 0,1 bis 3 Gew.-%, bezogen auf das Gesamtgewicht der Formulierung, verwendet.

Es liegt in der Natur von pharmazeutischen Hilfsstoffen, dass diese teilweise mehrere Funktionen in einer pharmazeutischen Formulierung wahrnehmen. Im Rahmen dieser Erfindung gilt zur unzweideutigen Abgrenzung daher bevorzugt die Fiktion, dass ein Stoff, der als ein bestimmter Hilfsstoff verwendet wird, nicht zeitgleich auch als weiterer pharmazeutischer Hilfsstoff eingesetzt wird. Beispielsweise wird Carrageenan, sofern als Ballaststoff (b) verwendet, dann nicht auch als Sprengmittel (c) verwendet (obwohl Carrageenan auch eine gewisse Sprengwirkung zeigt).

Es ist ein Vorteil der vorliegenden Erfindung, dass auf Feuchtestabilisatoren verzichtet werden kann. Die erfindungsgemäße pharmazeutische Zusammensetzung enthält bevorzugt keine Feuchthaltemittel, ausgewählt aus Glucose, Glucosederivaten und Zuckeralkoholen. Insbesondere enthält die erfindungsgemäße Zusammensetzung kein Feuchthaltemittel ausgewählt aus Isomalt, Xylitol, Sorbitol, Polydextrose, Dextrose oder Gemische daraus.

Die erfindungsgemäße pharmazeutische Zusammensetzung kann zu unterschiedlichen oralen Darreichungsformen verarbeitet werden. Bevorzugt wird sie zu Tabletten verpresst. In einer bevorzugten Ausführungsform liegt die erfindungsgemäße Zusammensetzung in Form einer Tablette vor, wobei die Tablette durch Direktkompression erhältlich ist. Ein geeignetes Direktkompressionsverfahren wird nachstehend näher beschrieben.

Alternativ kann die erfindungsgemäße Zusammensetzung (gegebenenfalls nach einem Granulierschritt) in Kapseln, Sachets oder Stickpacks abgefüllt werden.

In einer bevorzugten Ausführungsform handelt es sich bei der erfindungsgemäßen pharmazeutischen Zusammensetzung oder bei den erfindungsgemäßen oralen Darreichungsformen um Zusammensetzungen bzw. Darreichungsformen mit modifizierter Freisetzung. Unter dem Begriff "modifizierte Freisetzung" wird im Rahmen dieser Erfindung eine verzögerte Freisetzung *(delayed release),* eine gestaffelte Freisetzung *(repeat action release),* eine hinhaltende Freisetzung *(prolonged release),* eine gleichmäßig hinhaltende Freisetzung *(sustained release)* oder eine lang ausgedehnte Freisetzung *(extended release)* verstanden. Bevorzugt handelt es sich um eine hinhaltende Freisetzung *(prolonged release).* Insbesondere weisen die erfindungsgemäßen Zusammensetzungen bzw. oralen Darreichungsformen eine Freisetzungsrate von weniger als 60 % Wirkstoff nach 24 Stunden auf, gemessen nach USP (paddle, 900 ml Testmedium in Phosphatpuffer bei pH 6,8 und 37 °C).

Bevorzugt findet die erfindungsgemäße pharmazeutische Formulierung in Form von Tabletten Verwendung. Gegenstand der Erfindung ist daher ein Verfahren zur Herstellung einer Tablette, enthaltend die erfindungsgemäße pharmazeutische Formulierung, umfassend die Schritte
(i) Vermischen von
   (a) Fesoterodin und/oder Fesoterodin-Metabolite,
   (b) Ballaststoffe. mit pharmazeutischen Hilfsstoffen,
   und gegebenenfalls weiteren pharmazeutischen Hilfsstoffen,
(ii) Kompression zu Tabletten, gegebenenfalls unter Zusatz weiterer pharmazeutischer Hilfsstoffe, und
(iii) gegebenenfalls Befilmung der Tabletten.

Alle vorstehend gegebenen Erläuterungen zu bevorzugten Ausführungsformen der erfindungsgemäßen Zusammensetzung (z.B. zu Art und Menge der Komponenten (a) und (b) sowie der weiteren pharmazeutischen Hilfsstoffe) finden auch beim erfindungsgemäßen Verfahren Anwendung. Neben dem erfindungsgemäßen Verfahren sind auch Tabletten, erhältlich durch das erfindungsgemäße Verfahren, Gegenstand dieser Erfindung.

Im Schritt (i) werden die Komponenten (a) und (b) sowie gegebenenfalls weitere (vorstehend beschriebene) pharmazeutische Hilfsstoffe vermischt. Die Vermischung kann in üblichen Mischern erfolgen. Beispielsweise kann die Vermischung in Zwangsmischern oder Freifallmischern erfolgen (z.B. mittels Turbula^{®} T 10B (Bachofen AG, Schweiz)). Die Mischzeit kann beispielsweise 1 bis 10 Minuten betragen.

Im Anschluss an das Mischen kann die resultierende Mischung gesiebt werden. Sieben ist im Allgemeinen ein Vorgang zur Erzielung einer homogenen Pulvermischung. Beispielsweise können Trommelsiebe, Vibrationssiebe oder Konus-Siebmaschinen (insbesondere Quadro Comil^{®}) verwendet werden. Bevorzugt werden Siebe mit einer Maschenweite von 150 bis 750 *µ*m, insbesondere 300 bis 600 *µ*m, verwendet.

In Schritt (ii) erfolgt eine Kompression zu Tabletten. Die Kompression kann mit im Stand der Technik bekannten Tablettiermaschinen erfolgen. Die Kompression erfolgt bevorzugt in Abwesenheit von Lösungsmitteln.

Beispiele für geeignete Tablettiermaschinen sind Exzenterpressen oder Rundlaufpressen. Beispielsweise kann eine Fette 102i^{®} (Fette GmbH, Deutschland) verwendet werden. Im Falle von Rundlaufpressen wird üblicherweise eine Presskraft von 2 bis 40 kN, bevorzugt von 2,5 bis 35 kN angewandt. Im Falle von Exzenterpressen wird üblicherweise eine Presskraft von 1 bis 20 kN, bevorzugt von 2,5 bis 10 kN, angewandt. Beispielsweise wird die Korsch^{®} EKO verwendet.

Verfahrensschritt (ii) erfolgt bevorzugt in Abwesenheit von Lösungsmitteln, insbesondere organischen Lösungsmitteln, d.h. als Trockenkompression.

Im optionalen Schritt (iii) des erfindungsgemäßen Verfahrens werden die Tabletten aus Schritt (ii) befilmt. Hierbei können die im Stand der Technik üblichen Verfahren zur Befilmung von Tabletten Anwendung finden.

Für die Befilmung werden bevorzugt makromolekulare Stoffe verwendet, beispielsweise modifizierte Cellulosen, Polymethacrylate, Polyvinylpyrrolidon, Polyvinylacetatphthalat, Zein und/oder Schellack.

Die Schichtdicke des Überzugs beträgt bevorzugt 2 bis 100 *µ*m, mehr bevorzugt 10 bis 80 µm.

Die Tablettierbedingungen werden im erfindungsgemäßen Verfahrens ferner bevorzugt so gewählt, dass die resultierenden Tabletten ein Verhältnis von Tablettenhöhe zu Gewicht von 0,005 bis 0,3 mm/mg, besonders bevorzugt 0,05 bis 0,2 mm/mg, aufweisen.

Ferner weisen die resultierenden Tabletten bevorzugt eine Härte von 50 bis 200 N, besonders bevorzugt von 80 bis 150 N, auf. Die Härte wird gemäß Ph.Eur. 6.0, Abschnitt 2.9.8, bestimmt.

Zudem zeigen die resultierenden Tabletten bevorzugt eine Friabilität von kleiner 5 %, besonders bevorzugt von kleiner 3 %, insbesondere kleiner 2 % auf. Die Friabilität wird gemäß Ph.Eur. 6.0, Abschnitt 2.9.7. bestimmt.

Schließlich weisen die erfindungsgemäßen Tabletten üblicherweise eine Gleichförmigkeit des Gehalts *(Content Uniformity)* von 90 bis 110 %, bevorzugt von 95 bis 105 %, insbesondere von 98 bis 102 % vom durchschnittlichen Gehalt auf. Die "Content Uniformity" wird gemäß Ph. Eur.6.0, Abschnitt 2.9.6. bestimmt.

Die vorstehenden Angaben zu Härte, Friabilität, Content Uniformity und Freisetzungsprofil beziehen sich hierbei bevorzugt auf die unbefilmte Tablette.

In einer alternativen Ausführungsform werden die erfindungsgemäßen Tabletten nicht mittels Direktkompression, sondern mittels Trockengranulation und anschließender Verpressung hergestellt.

Ein Aspekt der vorliegenden Erfindung betrifft daher ein Trockengranulier-Verfahren umfassend die Schritte
(i-1) Vermischen von Fesoterodin (a) mit einem Ballaststoffen (b) und gegebenenfalls weiteren pharmazeutischen Hilfsstoffen;
(i-2) Kompaktierung zu einer Schülpe;
(i-3) Granulierung der Schülpe;
(ii) Kompression der resultierenden Granulate zu Tabletten, gegebenenfalls unter Zusatz weiterer pharmazeutischer Hilfsstoffe; und
(iii) gegebenenfalls Befilmung der Tabletten.

Im Schritt (i-2) des erfindungsgemäßen Verfahrens wird das Gemisch aus Schritt (i) zu einer Schülpe kompaktiert. Hierbei handelt es sich um eine Trockenkompaktierung, d.h. die Kompaktierung erfolgt bevorzugt in Abwesenheit von Lösungsmitteln, insbesondere in Abwesenheit von organischen Lösungsmitteln. Die Kompaktierung wird bevorzugt in einem Walzengranulator durchgeführt. Die Walzkraft beträgt üblicherweise 5 bis 70 kN/cm, bevorzugt 10 bis 60 kN/cm, mehr bevorzugt 15 bis 50 kN/cm. Die Spaltbreite des Walzgranulators beträgt beispielsweise 0,8 bis 5 mm, bevorzugt 1 bis 4 mm, mehr bevorzugt 1,5 bis 3 mm, insbesondere 1,8 bis 2,8 mm.

In Schritt (i-3) des Verfahrens wird die Schülpe granuliert. Die Granulierung kann mit im Stand der Technik bekannten Verfahren erfolgen. Beispielsweise erfolgt die Granulierung mit dem Gerät Comil^{®} U5 (Quadro Engineering, USA). Weiterhin werden die Granulierbedingungen bevorzugt so gewählt, dass die resultierenden Granulate eine Schüttdichte von 0,2 bis 0,85 g/ml, mehr bevorzugt 0,3 bis 0,8 g/ml, insbesondere 0,4 bis 0,7 g/ml, aufweisen. Der Hausner-Faktor liegt üblicherweise im Bereich von 1,03 bis 1,3, mehr bevorzugt von 1,04 bis 1,20 und insbesondere von 1,04 bis 1,15. Unter "Hausner-Faktor" wird hierbei das Verhältnis von Stampfdichte zu Schüttdichte verstanden.

In einer bevorzugten Ausführungsform erfolgt die Granulierung in einer Siebmühle. In diesem Fall beträgt die Maschenweite des Siebeinsatzes üblicherweise 0,1 bis 5 mm, bevorzugt 0,5 bis 3 mm, mehr bevorzugt 0,75 bis 2 mm, insbesondere 0,8 bis 1,8 mm.

Die erfindungsgemäßen Zusammensetzungen und oralen Darreichungsformen werden bevorzugt zur Behandlung der überaktiven Blase verwendet. Gegenstand der Erfindung ist somit die Verwendung von pflanzlichen Ballaststoffen, ausgewählt aus Alginate, Gelatine, Agar, Gummi arabicum, Traganth, Xanthan und Carrageenan, zur Herstellung einer pharmazeutischen Formulierung mit modifizierter Freisetzung zur Behandlung der überaktiven Blase. Anders ausgedrückt, Gegenstand der Erfindung ist ferner eine pharmazeutische Zusammensetzung mit modifizierter Freisetzung, enthaltend pflanzliche Ballaststoffen, ausgewählt aus Alginaten, Gelatine, Agar, Gummi arabicum, Traganth, Xanthan und Carrageenan, zur Behandlung der überaktiven Blase. Alle vorstehend gegebenen Erläuterungen zu bevorzugten Ausführungsformen der erfindungsgemäßen Zusammensetzung (z.B. zu Art und Menge der Komponente (b) sowie der weiteren pharmazeutischen Hilfsstoffe) finden auch bei der erfindungsgemäßen Verwendung Anwendung.

Die Erfindung soll anhand der folgenden Beispiele veranschaulicht werden.

### BEISPIELE

### Beispiel 1: Direktverpressung

Zur Herstellung von 200 Tabletten wurden 1,6 g Fesoterodinfumarat, 30,0 g Agar, 31,5 g Dextrin und 0,6 g Talkum eingewogen und 15 Minuten gemischt (Turbula^{®} T10B). Danach wurden 0,3 g Natriumstearylfumarat und zugegeben und wiederum 5 Minuten zusammen mit den anderen Stoffen gemischt (Turbula^{®} T10B).

Die Tabletten wurden zu 320 mg auf einer handelsüblichen Exzenterpresse (Korsch^{®} EKO) mit einer Form, die 12,5 x 6,5 mm betrug, verpresst.

### Beispiel 2: Direktverpressung

4 g Fesoterodinfumarat wurden mit 38,75 g Agar und 36,38 g Calciumphosphat, 10 Minuten gemischt (Turbula^{®} T10B). Die Mischung wurde über ein 500 *µ*m Sieb gegeben, 0,5 g Talkum und 0,38 g Natriumstearylfumarat zugegeben und es wurde die Mischung weitere 5 Minuten gemischt.

Aus der fertigen Mischung wurden 250 Tabletten ä 320 mg auf einer Exzenterpresse (Korsch^{®} EKO) verpresst.

### Beispiel 3: Trockengranulierung

Zur Herstellung von 160 Tabletten wurden 1,28 g Fesoterodinfumarat und 25,6 g Agar mit 2,5 g Wasser in einer Fantaschale unter Rühren granuliert.

Nach einer Trocknung von einer Stunde bei 40°C wurde die Mischung gesiebt (Comil^{®} U5) und anschließend eine weitere Stunde getrocknet.

23,4 g Dextrin und 0,48 g Talkum wurden zugegeben und die gesamte Mischung wurde 10 Minuten gemischt. Zuletzt wurden 0,24 g Natriumstearylfumarat und 0,24 g Natriumcarboxymethylstärke zugegeben, 3 Minuten gemischt und anschließend auf einer Exzenterpresse zu Tabletten mit 320 mg verpresst, wobei die Länge 12,5 mm und die Breite 6,5 mm betrug.

## Patentansprüche

1. Pharmazeutische Zusammensetzung, enthaltend
(a) Fesoterodin und/oder Fesoterodin-Metabolite und
(b) Ballaststoffe,
wobei das Gewichtsverhältnis der Komponenten (a) : (b) im Bereich von 1 : 50 bis 1 : 2 liegt.

2. Pharmazeutischen Zusammensetzung nach Anspruch 1, wobei es sich bei dem Wirkstoff (a) um Fesoterodin-Hydrogenfumarat, Fesoterodin-Fumarat, Fesoterodin-Tartrat, Fesoterodin-5-HM-Hydrogenfumarat, Fesoterodin-5-HM-Fumarat, Fesoterodin-5-HM-Tartrat und/oder Fesoterodin-5-HM-Hydrogentartrat handelt.

3. Pharmazeutischen Zusammensetzung gemäß Anspruch 1 oder 2, wobei es sich bei der Komponente (b) um pflanzliche Ballaststoffe, bevorzugt um pflanzliche Ballaststoffe mit Geliervermögen handelt.

4. Pharmazeutische Zusammensetzung gemäß einem der Ansprüche 1 bis 3, wobei die Ballaststoffe (b) frei von Cellulose oder Cellulosederivaten sind.

5. Pharmazeutische Zusammensetzung gemäß einem der Ansprüche 1 bis 4, wobei die Ballaststoffe eine Gelstärke von 50 bis 300 g aufweisen.

6. Pharmazeutische Zusammensetzung gemäß einem der Ansprüche 1 bis 5, wobei die Ballaststoffe (b) ausgewählt sind aus Alginaten, Gelatine, Agar, Gummi Arabicum, Traganth, Xanthan und Carrageenan.

7. Pharmazeutische Zusammensetzung gemäß einem der Ansprüche 1 bis 6, wobei Agar als Ballaststoff (b) verwendet wird.

8. Pharmazeutische Zusammensetzung gemäß einem der Ansprüche 1 bis 7, wobei die Zusammensetzung frei von Feuchthaltemitteln, ausgewählt aus Glucose, Glucosederivaten und Zuckeralkoholen, ist.

9. Pharmazeutische Zusammensetzung gemäß einem der Ansprüche 1 bis 8, enthaltend
a) 0,1 bis 20 Gew.-% Fesoterodin und/oder Fesoterodin-Metabolite;
b) 0,5 bis 80 Gew.-% Ballaststoffe;
c) 0 bis 15 Gew.-% Sprengmittel; und
d) 0 bis 80 Gew.-% Tablettiermittel.

10. Pharmazeutische Zusammensetzung gemäß einem der Ansprüche 1 bis 9 in Form einer Tablette, wobei die Tablette durch Direktkompression erhältlich ist.

11. Pharmazeutische Zusammensetzung gemäß einem der Ansprüche 1 bis 10, wobei es sich um eine Zusammensetzung mit modifizierter Freisetzung handelt.

12. Verfahren zur Herstellung von Tabletten, umfassend die Schritte:
(i) Vermischen von
(a) Fesoterodin und/oder Fesoterodin-Metabolite,
(b) Ballaststoffen. mit pharmazeutischen Hilfsstoffen,
und gegebenenfalls weiterer pharmazeutischer Hilfsstoffe,
(ii) Kompression zu Tabletten, gegebenenfalls unter Zusatz weiterer pharmazeutischer Hilfsstoffe, und
(iii) gegebenenfalls Befilmung der Tabletten.

13. Tabletten, erhältlich nach einem Verfahren gemäß Anspruch 12.

14. Tablette mit einer Friabilität von kleiner 3 %, mit einer Gleichförmigkeit des Gehalts von 95 bis 105 % und mit einer Härte von 50 bis 180 N, enthaltend eine pharmazeutische Zusammensetzung gemäß einem der Ansprüche 1 bis 12.

15. Verwendung von pflanzlichen Ballaststoffen, ausgewählt aus Alginaten, Gelatine, Agar, Gummi Arabicum, Traganth, Xanthan und Carrageenan, zur Herstellung einer pharmazeutischen Formulierung mit modifizierter Freisetzung zur Behandlung der überaktiven Blase.
